# EUROPEAN PATENT SPECIFICATION

(11) **EP 1 477 477 B1**
(45) Date of publication and mention of the grant of the patent: **22.12.2010**
(21) Application number: 03705138.0
(22) Date of filing: 14.02.2003
(51) Int. Cl.: C07C 403/24, C07C 403/22, C07C 317/24, C07F 9/54

(54) **PROCESS FOR PREPARATION OF CAROTENOIDS**
VERFAHREN ZUR HERSTELLUNG VON CAROTENOIDEN
PROCEDE DE PREPARATION DE CAROTENOIDES

(30) Priority: 19.02.2002 JP 2002041247; 29.11.2002 JP 2002347572
(43) Date of publication of application: 17.11.2004
(73) Proprietor: Sumitomo Chemical Company, Limited, Tokyo 104-8260 (JP)
(72) Inventor: SEKO, Shinzo, Toyonaka-shi, Osaka 560-0003 (JP); KONYA, Naoto, Osaka-shi, Osaka 547-0027 (JP); TAKAHASHI, Toshiya, Kawanishi-shi, Hyogo 666-0136 (JP)
(74) Representative: Henkel, Feiler & Hänzel
(86) International application number: PCT/JP2003/001520
(87) International publication number: WO 2003/070698

(56) References cited:
- EP-A1- 1 092 709
- EP-A1- 1 199 303
- DE-A- 19 926 972
- US-A- 5 237 102

## Description

### Technical Field

The present invention relates to a process for preparation of carotenoids which are important in the fields of medicine, feed additives, food additives and the like, and to intermediates thereof.

### Background Art

Heretofore, as synthetic methods of carotenoides which are symmetric C40 compounds, there are a coupling method of two molecules of a C19 compound with a C2 compound, a coupling method of two molecules of a C15 compound with a C10 compound, a coupling method of two molecules of a C13 compound with a C14 compound (see, e.g., Helv. Chim. Acta, Vol. 39, 249 (1956)), and a coupling method of two molecules of a C20 compound (see, e.g., Pure & Appl. Chem., Vol. 63, 35 (1991), Japanese Patent No. 2506495, JP 8-311020 A). However, they can not necessarily be said to be industrial methods having high practical value because they required a multi-stage step, their intermediates are unstable, etc.

US-A-5 237 102 discloses a process for readily producing β-carotene in which a sulfone compound represented by the general formula (1), (2) or (3) is reacted with an alkali, 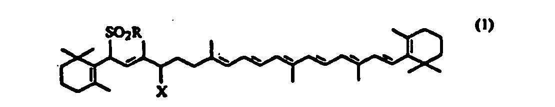 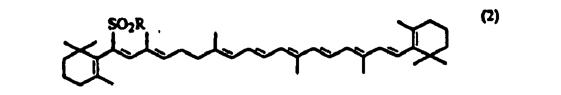 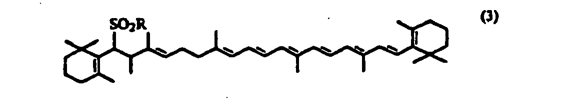 wherein R represents a phenyl group which may be substituted, R' a protecting group of acetal type and X a halogen atom.

EP-A-1 092 709 discloses a process for producing a sulfone derivative of formula (1): 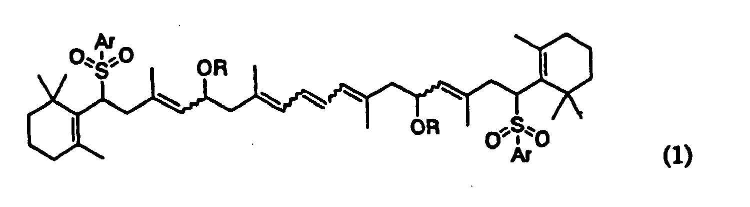 wherein Ar represents an aryl group which may be substituted, R represents a lower alkyl group and the wavy line depicted by " 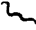 " indicates a single bond and a stereochemistry relating to a double bond bound therewith is E or Z or a
mixture thereof, which process is characterized by reacting an aldehyde derivative of formula (2): 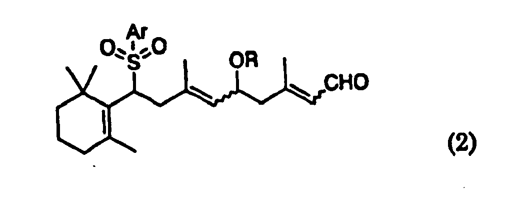 with a phosphonium salt of formula (3): 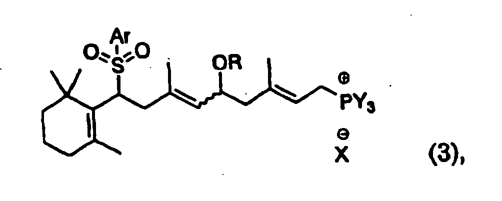 in the presence of a base; a sulfone derivative of formula (1) and a process for producing a β-carotene using sulfone derivative of formula (1).

### Disclosure of Invention

The present inventors have studied intensively so as to develop a process for industrially advantageous preparation of carotenoids without passing through unstable intermediates. As a result, the present inventors have found a method wherein a carotenoid is derived from coupling of two molecules of a C15 compound, which can be readily synthesized from a relatively cheap C10 compound such as myrcene or linalool, with a C10 compound to obtain a relatively stable C40 disulfone compound, followed by reacting the resultant compound with a basic compound. Thus, the present inventors have achieved the present invention.

That is, according to the present invention, there is provided:
A process for preparation of a halogen compound represented by the general formula (13): 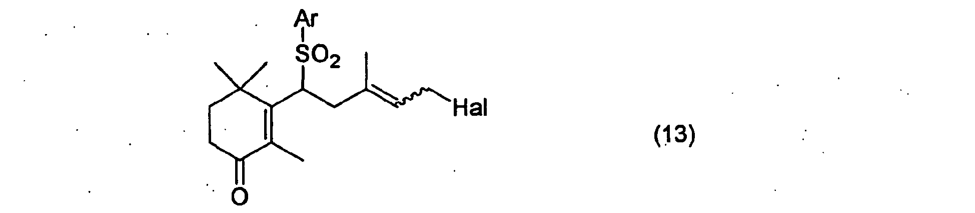 wherein Ar, Hal and the wavy line are as defined hereinafter, which comprises the steps of:
reacting a cyclic sulfone compound represented by the general formula (8): 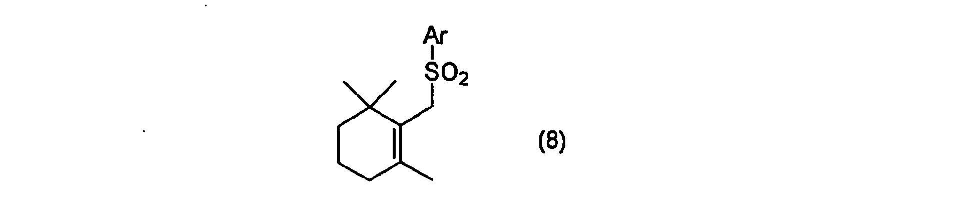 wherein Ar is an optionally substituted aryl group, with a N-halogeno compound in the presence of water to obtain a cyclic ketosulfone compound represented by the general formula (9): 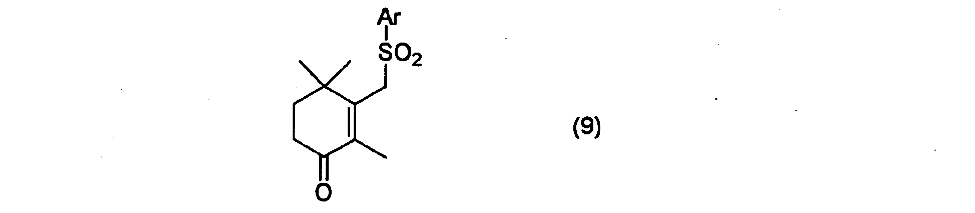 wherein Ar is as defined above, reacting the cyclic ketosulfone compound with an allyl halide represented by the general formula (10) : 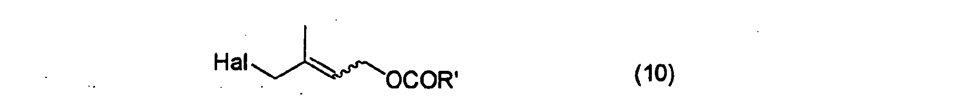 wherein Hal is a halogen; R' is a straight- or branched-chain C₁-C₅ alkyl group; and the wavy line represents either of E/Z geometrical isomers or a mixture thereof, in the presence of a base to obtain an ester compound represented by the general formula (11): 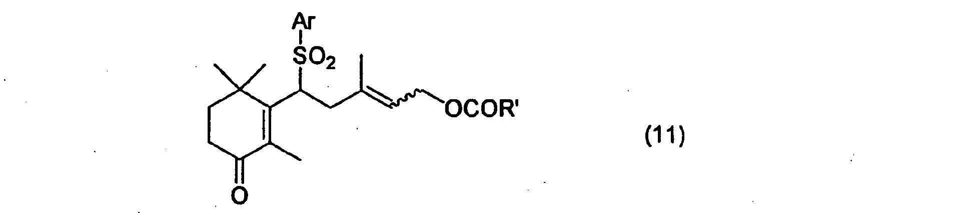 wherein Ar, R' and the wavy line are as defined above, hydrolyzing the ester compound to obtain an alcohol compound represented by the general formula (12): 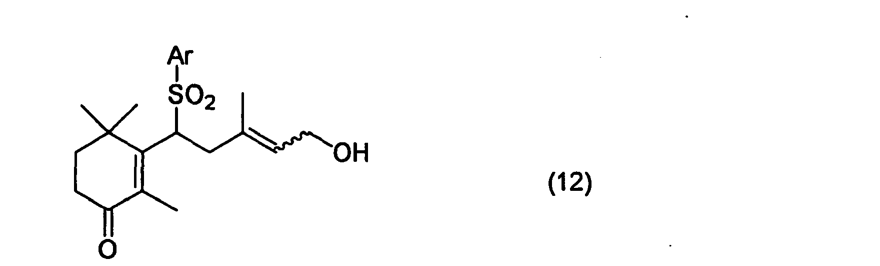
wherein Ar and the wavy line are as defined above, and reacting the alcohol compound with an halogenating agent; and

A process for preparation of a carotenoid represented by the general formula (4): 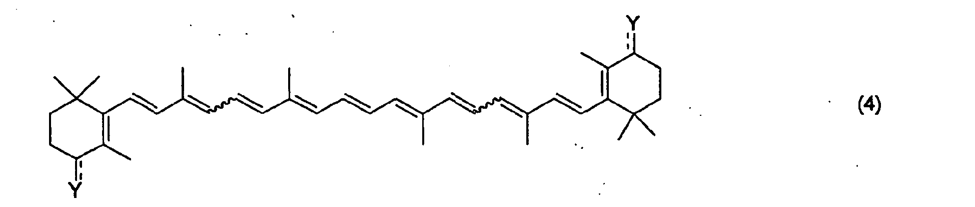 wherein Y and each wavy line are as defined hereinafter, which comprises the steps of:
reacting a sulfone compound represented by the general formula (6): 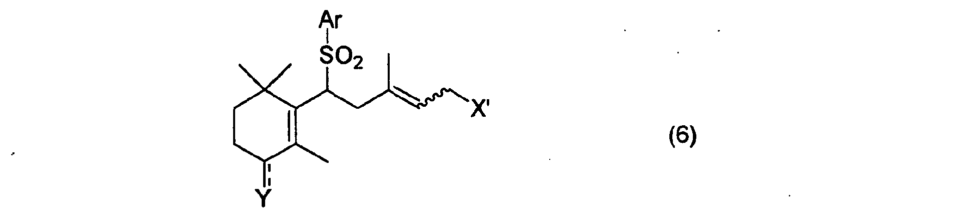 wherein Ar is an optionally substituted aryl group; X' is a hydroxy group or a halogen; Y is a hydrogen atom or an oxo group; and the wavy line represents either of E/Z geometrical isomers or a mixture thereof, with a triarylphosphine represented by the general formula (7):

   PAr'₃ (7)

   wherein Ar' is an optionally substituted aryl group, or a hydrohalogenic acid salt or sulfonate thereof to obtain a phosphonium salt represented by the general formula (2): 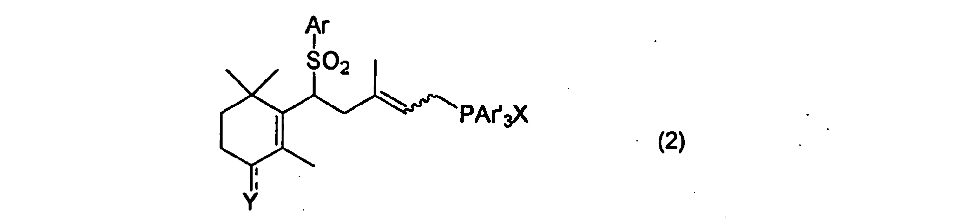 wherein Ar and Ar' are as defined above; X is a halogen or -OSO₂R" group, wherein R" is an optionally substituted aryl group, a straight- or branched-chain C₁-C₅ alkyl group, or a trifluoromethyl group; and Y and the wavy line are as defined above, reacting the phosphonium salt with a C10 dialdehyde represent by the formula (5): 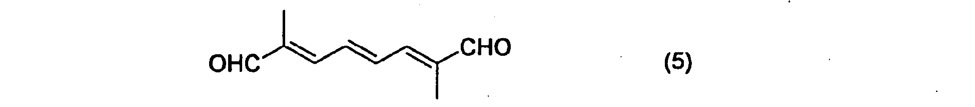 in the presence of a base to obtain a disulfone compound represented by the general formula (3): 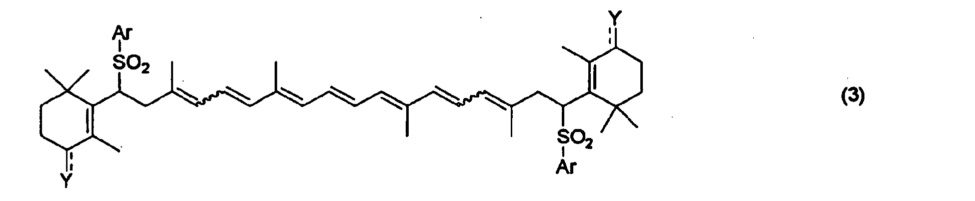 wherein Ar, Y and each wavy line are as defined above, and reacting the disulfone compound with a basic compound; and

A ketosulfone compound represented by the general formula (1) : 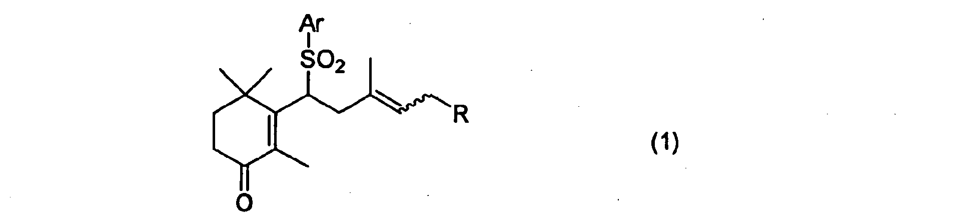 wherein Ar is as defined above; R is a hydroxy group or -OCOR' group or a halogen, wherein R' is a straight- or branched-chain C₁-C₅ alkyl group; and the wavy line is either of E/Z geometric isomers or a mixture thereof; and

A phosphonium salt represented by the general formula .(2) : 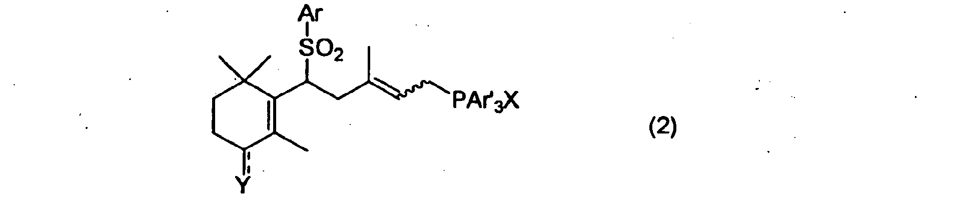 wherein each of Ar and Ar' is an optionally substituted aryl group; X is a halogen or -OSO₂R" group, wherein R" is an optionally substituted aryl group, a straight- or branched-chain C₁-C₅ alkyl group, or a trifluoromethyl group; Y is a hydrogen atom or an oxo group; and the wavy line represents either of E/Z geometric isomers or a mixture thereof; and

A disulfone compound represented by the general formula (3): 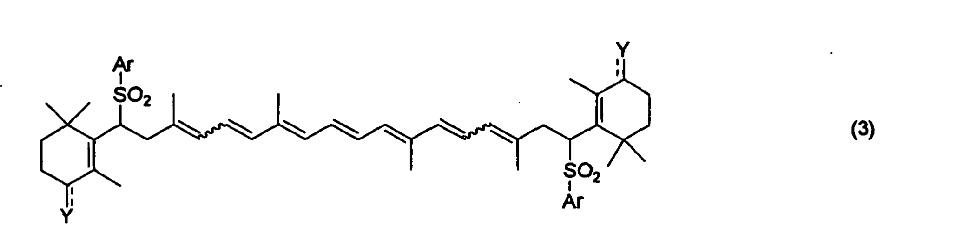 wherein Ar is an optionally substituted aryl group; Y is a hydrogen atom or an oxo group; each wavy line represents either of E/Z geometric isomers or a mixture thereof.

### Embodiment for Performing the Invention

Hereinafter, the present invention will be illustrated in detail.

Examples of the aryl group of the optionally substituted aryl group represented by the substituents Ar, Ar' and R" in the compounds represented by the formulas (1), (2), (3), (6), (7), (8), (9), (11), (12) and (13) include a phenyl group, a naphthyl group, etc. As the substituent, there are a C₁-C₅ alkyl group, a C₁-C₅ alkoxy group, a halogen, a nitro group, and the like. Specific examples thereof include phenyl, naphthyl, o-tolyl, m-tolyl, p-tolyl, o-methoxyphenyl, m-methoxyphenyl, p-methoxyphenyl, o-chlorophenyl, m-chlorophenyl, p-chlorophenyl, o-bromophenyl, m-bromophenyl, p-bromophenyl, o-iodophenyl, m-iodophenyl, p-iodophenyl, o-fluorophenyl, m-fluorophenyl, p-fluorophenyl, o-nitrophenyl, m-nitrophenyl, p-nitrophenyl, and the like.

Further, the straight- or branched-chain lower alkyl represented by the substituents R' and R" in the compounds represented by the general formula (1), (2), (10) and (11) is the group having 1 to 5 carbon atoms, and specific examples thereof include methyl group, ethyl group, n-propyl group, isopropyl group, n-butyl group, isobutyl group, t-butyl group, n-pentyl group, and the like.

Specifically, the halogen represented by the substituents X, X' and Hal in the compounds represented by the general formula (2), (6), (10) and (13) includes a chlorine atom, a bromine atom and an iodine atom.

The cyclic ketosulfone compound (9) can be obtained by subjecting the cyclic sulfone compound (8) to an oxidation reaction. As the oxidizing agent, metallic oxidizing agents such as chromium, manganese, selenium, and the like have been heretofore known (JP 2000-80073 A). A N-halogeno compound can also be used as the oxidizing agent in the presence of water. Examples of the N-halogeno compound include N-halogenosuccinimide, trihalogenoisocyanuric acid, 1,3-dihalogeno-5,5-dimethylhydantoin, and the like. Specifically, there are N-bromosuccinimide, N-chlorosuccinimide, N-iodosuccinimide, tribromoisocyanuric acid, trichloroisocyanuric acid, triidoisocyanuric acid, 1,3-dibromo-5,5-dimethylhydantoin,
1,3-dichloro-5,5-dimethylhydantoin,
1,3-diiodo-5,5-dimethylhydantoin, chloroamine, chloramine T, chloramine B, N-chlorourea, N-bromoacetamide, and the like.

The amount of the N-halogeno compound to be used is usually 1 to 10 moles preferably 1 to 4 moles to 1 mole of the cyclic sulfone compound (8).

Usually, an organic solvent is used in the above oxidation reaction. Examples of the solvent include aprotic polar solvents such as acetonitrile, N,N-dimethylformamide, dimethyl sulfoxide, N,N-dimethylacetamide, hexamethylphosphoric triamide, etc.; ether solvents such as 1,4-dioxane, tetrahydrofuran, etc.; hydrocarbon solvents such as n-hexane, cyclohexane, n-pentane, n-heptane, toluene, xylene, etc.; halogenated solvents such as chloroform, dichloromethane, 1,2-dichloroethane, monochlorobenzene, o-dichlorobenzene, etc.; and the like. These solvents can be used alone or in a combination of two or more thereof.

The above oxidation reaction is performed by addition of water to the above organic solvent. The amount of water to be added is 1 equivalent or more to the cyclic sulfone compound (8) and, usually, 5 to 50% by weight based on the solvent to be used.

The reaction temperature is usually within the range of 0°C to the boiling point of the solvent to be used. Further, the reaction time varies depending upon the kind of oxidizing agent used in the reaction and the reaction temperature, but is usually within the range of about 1 hour to 24 hours.

After the reaction, the cyclic ketosulfone compound (9) can be obtained by performing a conventional post-treatment operation. Further, if necessary, it can be purified by silica gel chromatography, etc.

The ester compound (11) in the present invention can be obtained by reacting the cyclic ketosulfone compound (9) with the allyl halide (10) in the presence of a base.

Examples of the base to be used in the above reaction include alkyllithium, Grignard reagents, alkali metal hydroxides, alkaline earth metal hydroxides, alkali metal hydrides, alkaline earth metal hydrides, alkali metal alkoxides, alkaline earth metal alkoxides, and the like. Specifically, there are n-butyllithium, s-butyllithium, t-butyllithium, methylmagnesium bromide, methylmagnesium chloride, ethylmagnesium bromide, ethylmagnesium chloride, sodium hydroxides potassium hydroxide, sodium-hydride, potassium hydride, sodium methoxide, potassium methoxide, sodium t-butoxide, potassium t-butoxide, and the like.

The amount of the base to be used is usually about 0.1 to 20 moles to 1 mole of the cyclic ketosulfone compound (9).

In some cases, a phase-transfer catalyst is preferably used to accelerate the reaction.

Examples of the phase-transfer catalyst include quaternary ammonium salts, quaternary phosphonium salts, sulfonium salts, and the like.

As the quaternary ammonium salts, there are ammonium halides having alkyl and/or aralkyl groups containing 1 to 24 carbon atoms. Examples thereof include tetramethylammonium chloride, tetraethylammonium chloride, tetrapropylammonium chloride, tetrabutylammonium chloride, tetrapentylammonium chloride, tetrahexylammonium chloride, tetraheptylammonium chloride, teteraoctylammonium chloride, tetrahexadecylammonium chloride, tetraoctadecylammonium chloride, benzyltrimethylammonium chloride, benzyltriethylammonium chloride, benzyltributylammonium chloride, 1-methylpyridinium chloride, 1-hexadecylpyridinium chloride, dimethylpyridinium chloride, trimethylcyclopropylammonium chloride, tetramethylammonium bromide, tetraethylammonium bromide, tetrapropylammonium bromide, tetrabutylammonium bromide, tetrapentylammonium bromide, tetrahexylammonium bromide, tetraheptylammonium bromide, tetraoctylammonium bromide, tetrahexadecylammonium bromide, tetraoctadecylammnoium bromide, benzyltrimethylammonium bromide, benzyltriethylammonium bromide, benzyltributylammonium bromide, 1-methylpyridinium bromide, 1-hexadecylpyridinium bromide, dimethypyridinium, bromide, trimethylcyclopropylammonium bromide, tetramethylammonium iodide, tetrabutylammonium iodide, tetraoctylammonium iodide, t-butylethyldimethylammonium iodide, tetradecyltrimethylammonium iodide, hexadecyltrimethylammonium iodide, octadecyltrimethylammonium iodide, benzyltrimethylammonium iodide, benzyltriethylammonium iodide, benzyltributylammonium iodide, and the like.

Examples of the quaternary phosphonium salt include tributylmethylphosphonium chloride, triethylmethylphosphonium chloride, methyltriphenoxyphosphonium chloride, butyltriphenylphosphonium chloride, tetrabutylphosphonium chloride, benzyltriphenylphosphonium chloride, hexadecyltrimethylphosphonium chloride, hexadecyltributylphosphonium chloride, hexadecyldimethylethylphosphonium chloride, tetraphenylphosphonium chloride, tributylmethylphosphonium bromide, triethylmethylphosphonium bromide, methyltriphenoxyphosphonium bromide, butyltriphenylphosphonium bromide, tetrabutylphosphonium bromide, benzyltriphenylphosphonium bromide, hexadecyltrimethylphosphonium bromide, hexadecyltributylphosphonium bromide, hexadecyldimethylethylphosphonium bromide, tetrapheylphosphonium bromide, tributylmethylphosphonium iodide, triethylmethylphosphonium iodide, methyltriphenoxyphosphonium iodide, butyltriphenylphosphonium iodide, tetrabutylphosphonium iodide, benzyltriphenylphosphonium iodide, hexadecyltrimethylphosphonium iodide, and the like.

Examples of the sulfonium salt include dibutylmethylsulfonium chloride, trimethylsulfonium chloride triethylsulfonium chloride, dibutylmethylsulfonium bromide, trimethylsulfonium bromide, triethylsulfonium bromide dibutylmethylsulfonium iodide, trimethylsulfonium iodide, triethylsulfonium iodide, and the like.

Among them, the preferred catalysts are the quaternary ammonium salts, in particular, ammonium halides having alkyl and/or aryl groups containing 1 to 24 carbon atoms.

Usually, the amount of the phase-transfer catalyst to be used is 0.01 to 0.2 mole, preferably 0.02 to 0.1 mole to 1 mole of the cyclic ketosulfone compound (9).

In the above reaction, usually, an organic solvent is used, and examples of the solvent include ether solvents such as diethyl ether, 1,4-dioxane, tetrahydrofuran, anisole, etc.; hydrocarbon solvents such as n-hexane, cyclohexane, n-pentane, n-heptane, toluene, xylene, etc.; or aprotic polar solvents such as N,N-dimethylformamide, dimethyl sulfoxide, N,N-dimethylacetamide, hexamethylphosphoric triamide, etc.

The reaction temperature is usually within the range of -78°C to the boiling point of the solvent to be used. Further, the reaction time varies depending on the kind of base and the catalyst used in the reaction and the reaction temperature, but is usually within the range of about 0.5 hour to 24 hours.

After the reaction, the ester compound (11) can be obtained by performing a conventional post-treatment operation. At this time, the alcohol compound (12) is sometimes formed in the post-treatment step.

The alcohol compound (12) can be readily derived from the above-obtained ester compound (11) by conventional hydrolysis. The hydrolysis reaction is not specifically limited and, for example, there is the method described in Comprehensive Organic Transformation, R.C. Larock, VCH publishers Inc., p 981 (1988) If necessary, the alcohol compound (12) thus obtained can be purified by silica gel chromatography, etc.

The halogen compound (13) of the present invention can be obtained by subjecting the alcohol compound (12) to a halogenation reaction.

Examples of the above halogenation reaction include a halogenation reaction using a halide of a group 4 transition metal, a halogenation reaction using a halide of sulfur, phosphorus or boron or an acid chloride, a halogenation reaction using these halogenating agents together with formamides, a halogenation reaction using hydrogen chloride, hydrochloric acid, hydrogen bromide, or hydrobromic acid, and the like.

Examples of the halide of the group 4 transition metal include titanium tetrachloride, titanium tetrabromide, titanium tetraiodide, dichlorotitanium isopropoxide, chlorotitanium triisopropoxide, zirconium tetrachloride, hafnium tetrachloride, and the like. Among them, the preferred halide is titanium tetrachloride and, in case of using titanium tetrachloride, preferably, it is used in the presence of an ether compound such as ethylene glycol dimethyl ether, etc. or a ketone compound such as acetone, methyl isobutyl ketone, etc.

Examples of the halide of sulfur, phosphorus or boron include thionyl chloride, thionyl bromide, phosphorus trichloride, phosphorus pentachloride, phosphorus oxychlordie, phosphorus tribromide, phosphorus pentabromide, phosphorus triiodide, boron trichloride, boron tribromide, and the like. Examples of the acid chloride include phosgene, oxalyl chloride, and the like. These halogenating agents can be used alone. Alternatively, they can be used in the presence of formamides such as N,N-dimethylformamide to form Vilsmeier agents, which can be utilized as the halogenating agents.

Usually, an organic solvent is used for the above reaction and examples thereof include ether solvents such as diethyl ether, 1,4-dioxane, tetrahydrofuran, anisole, etc.; hydrocarbon solvents such as n-hexane, cyclohexane, n-pentane, n-heptane, toluene, xylene, etc.; halogenated solvents such as chloroform, dichloromethane, 1,2-dichloroethane, monochlorobenzene, etc.; ketone solvents such as acetone, methyl isopropyl ketone, etc.; or aprotic polar solvents such as acetonitrile, N, .N-dimethylformamide, dimethyl sulfoxide, N,N-dimethylacetamide, hexamethylphosphoric triamide, etc.

Usually, the reaction temperature can arbitrarily be selected within the range of -78°C to the boiling point of the solvent to be used, preferably within the range of about -20°C to 80°C. Further, the reaction time varies depending on the kind of halogenating agent and the reaction temperature but, usually, is within the range of about 0.5 hour to 24 hours.

After the reaction, the halogen compound (13) can be obtained by performing a conventional post-treatment operation.

The phosphonium salt (2) of the present invention can be obtained by reacting the sulfone compound (6) with the triarylphosphine (7) or a hydrohalogenic acid salt or sulfonate thereof.

Examples of the triarylphosphine to be used in the above triarylphosphine-formation reaction include triphenylphosphine, tri-o-tolylphosphine, tri-m-tolylphosphine, tri-p-tolylphosphine, tris(3-methoxyphenyl)phosphine, tris(4-methoxyphenyl)phosphine, and the like. Examples of the hydrohalogenic acid salt or sulfonate thereof include hydrochloride, hydrobromide, hydroiodide, methanesulfonate, ethanesulfonate, benzenesulfonate, p-toluenesulfonate, trifluoromethanesulfonate, and the like.

The amount of the triarylphosphine or a hydrohalogenic acid salt or sulfonate thereof to be used is usually about 0.7 to 2 moles to 1 mole of the sulfone compound (6).

Usually, an organic solvent is used for the above triarylphosphine-formation reaction. Examples of the solvent include ether solvents such as diethyl ether, tetrahydrofuran, dimethoxyethane, 1,4-dioxane, anisole, etc.; aprotic polar solvents such as N,N-dimethylformamide, dimethyl sulfoxide, N,N-dimethylacetamide, sulfolane, hexamethylphosphoric triamide, etc.; hydrocarbon solvents such as n-hexane, cyclohexane, n-pentane, n-heptane, benzene, toluene, xylene, etc.; ketone solvents such as acetone, diisopropyl ketone, methyl isobutyl ketone, etc.; halogenated solvents such as chloroform, dichloromethane, 1,2-dichloroethane, monochlorobenzene, o-dichlorobenzene, α,α,α-trifluorotoluene, etc.; and alcohol solvents such as methanol, ethanol, etc.

Usually, the reaction temperature is within the range of about 10°C to 50°C. The reaction time is about 1 hour to 24 hours. After the reaction, the resultant phosphonium salt (2) can be isolated, but it can also be used in the next step without isolation.

In case of the sulfone compound represented by the above general formula (6) wherein Y is a hydrogen atom and X' is a hydroxy group, the compound can be produced by the process described in EP 1 199 303 A1. When Y is a hydrogen atom and X' is a halogen, the sulfone compound can be produced by subjecting the corresponding sulfone compound wherein X is a hydrogen atom and X' is a hydroxy group to the same halogenation reaction as that described above.

The disulfone compound (3) of the present invention can be obtained by reacting the above phosphonium compound (2) with the C10 dialdehyde (5) in the presence of a base. The amount of the phosphonium compound (2) to be used is about 2 to 3 moles to 1 mole of the C10 dialdehyde (5).

As the base to be used in the above reaction, any base can be used in so far as it can be used for a conventional Wittig reaction. Specifically, for example, there are alkali metal alkoxides such as potassium methoxide, potassium ethoxide, potassium n-butoxide, potassium t-butoxide, sodium methoxide, sodium ethoxide, sodium n-butoxide, sodium t-butoxide, etc.; alkali metal hydroxides such as potassium hydroxide, sodium hydroxide; lithium hydroxide, etc.; and the like. Alternatively, epoxides such as ethylene oxide, 1,2-butene oxide, etc., can be used instead of the base.

The amount of such a base is usually about 1 to 5 moles to 1 mole of the phosphonium salt (2). Usually, an organic solvent is used for the above reaction. As these solvents, there are hydrocarbon solvents such as n-hexane, cyclohexane, n-pentane, n-heptane, toluene, xylene, etc.; ether solvents such as diethyl ether, tetrahydrofuran, dimethoxyethane, anisole, etc.; halogenated solvents such as chloroform, dichloromethane, 1,2-dichloromethane, monochlorobenzene, o-dichlorobenzene, etc.; or aprotic polar solvents such as N,N-dimethylformamide, dimethyl sulfoxide, N,N-dimethylacetamide, hexamethylphosphoric triamide, etc. Sometimes, a mixed solvent thereof with water can also be used.

Usually, the reaction temperature is within the range of about -10°C to 150°C, preferably about 0°C to 100°C. The reaction time varies depending on the kind of solvent used and the reaction temperature but, usually, it is within about 5 hours to 48 hours. After the reaction, the disulfone compound (3) can be obtained by performing a conventional post-treatment operation. However, if necessary, it can be purified by extraction, washing, various chromatographic techniques, and the like.

The carotenoid (4) can be derived from the resultant disulfone compound (3) by reaction thereof with basic compound. Examples of the basic compound to be used in the above reaction include alkali metal hydroxides, alkali metal hydrides, and alkali metal alkoxides. Specifically, there are, for example, sodium hydroxide, potassium hydroxide, lithium hydroxide, sodium hydride, potassium hydride, sodium methoxide, potassium methoxide, sodium ethoxide, potassium ethoxide, sodium t-butoxide, potassium t-butoxide, etc. The amount of the basic compound is usually about 2 to 30 moles, preferably about 4 to 25 moles to 1 mole of the disulfone compound (3).

Usually, an organic solvent is used for the above reaction. Examples of the solvent include ether solvents such as diethyl ether, tetrahydrofuran, 1,4-dioxane, anisole, etc.; hydrocarbon solvents such as n-hexane, cyclohexane, n-pentane, n-heptane, toluene, xylene, etc.; alcohol solvents such as methanol, ethanol, isopropanol, t-butanol, etc.; and aprotic polar solvents such as acetonitrile, N,N-dimethylformamide, dimethyl sulfoxide, N,N-dimethylacetamide, hexamethylphosphoric triamide, etc.

Usually, the reaction temperature is within -78°C to the boiling point of the solvent to be used. Further, the reaction temperature varies depending on the kind of basic compound used in the reaction and the reaction temperature, but it is usually within the range of about 1 hour to 24 hours. After the reaction, the carotenoid (4) can be obtained by performing a conventional post-treatment operation. If necessary, it can be purified by crystallization, various chromatographic techniques, and the like. However, since the carotenoid (4) is liable to be oxidized, desirably, these operations are performed under an atmosphere of inert gas such as nitrogen, argon, etc., and an antioxidant such as BHT, etc., is added to the solvent to be used.

The above starting material, i.e., the cyclic sulfone compound (8) can be synthesized from myrcene or linalool by a known method (e.g., Chem. Lett., 479 (1975); Japanese Patent 2558275, etc.).

The allyl halide (10) can be synthesized by a known method (e.g., JP 6-345689 A; EP 1 231 197 A1, etc.).

The C10 dialdehyde (5) can be synthesized by a known method (e.g., DE 1 092 472; Pure & Appl. Chem. Vol. 47, 173 (1976), etc.).

### Examples

Hereinafter, the present invention will be further illustrated by the following Examples, but they are not to be construed to limit.the scope of the present invention.

### Example 1 (Reference Example)

The cyclic sulfone compound (XIV) (18.28 g, 62.5 mmol) was dissolved in a mixed solvent of acetonitrile (300 ml) and water (30 ml). To this solution was added N-bromosuccinimide (16.7 g, 93.8 mmol) at room temperature. After stirring the mixture at the same temperature for 30 minutes, N-bromosuccinimide (11.1 g, 62.4 mmol) was further added thereto and the mixture was stirred for 7 hours. After addition of N,N-diethylaniline (25 ml), an aqueous 5% sodium sulfite solution (150 ml) was added and the mixture was extracted three times with diethyl ether. The resultant organic layer was washed with successive, 1 M hydrochloric acid, water and saturated saline, dried over MgSO₄ and concentrated to obtain a crude product. The crude product was purified by silica gel column chromatography to obtain the cyclic ketosulfone compound (I) in a yield of 49%.

### Example 2

Sodium t-butoxide (0.59 g, 6 mmol) was dissolved in DMF (15 ml) and to the solution was added a solution of the cyclic ketosulfone compound (I) (1.532 g, 5 mmol) in DMF (10 ml) all at once at -20°C. After one minute, a solution of the allyl halide (II) (1.2983 g, 6 mmol) in DMF (10 ml) was added dropwise thereto over one minute and the mixture was stirred at the same temperature for 2 hours. After completion of the reaction, an aqueous saturated NH₄Cl solution was added thereto and the mixture was extracted with ethyl acetate. The resultant organic layer was washed with water and saturated saline, dried over MgSO₄ and concentrated to obtain a crude product. The crude product was purified with silica gel column chromatography to obtain the ester compound (III) (1.414 g, 3.27 mmol, yield: 65.4.%).
¹H-NMR: δ (CDCl₃) 0.98 (3H), 1.27 (3H), 1.34 (3H), 1. 70-2.10 (2H), 2.04 (3H), 2.17 (3H), 2.47 (3H), 2.40-2.65 (2H), 2.65-2.85 (1H), 2.95-3.20 (1H), 4.00-4.20 (1H), 4.20-4.60 (2H), 5.30-5.50 (1H), 7.30-7.50 (2H), 7.70-7.90 (2H)

### Example 3

The ester compound (III) (1.3715 g, 3.2 mmol) was dissolved in methanol (20 ml), an aqueous 2 M NaOH solution (2 ml, 6 mmol) was added thereto at room temperature and the mixture was stirred as such for 5.5 hours. After completion of the reaction, extraction was performed by addition of water and Et₂O. The resultant organic layer was washed with saturated saline, dried over MgSO₄, and concentrated to obtain the alcohol compound (IV) (1.1366 g, 2.91 mmol, yield: 91%).
¹H-NMR: δ (CDCl₃) 1.00 (3H), 1.26(3H), 1.33(3H), 1.60-2.05 (3H), 2.17 (3H), 2.46 (3H), 2.30-2.60 (2H), 2.65-2.75 (1H), 2.95-3.10 (1H), 4.00-4.10 (1H), 4.10-4.30 (2H), 5.30-5.60 (1H), 7.30-7.40 (2H), 7.70-7.90 (2H)

### Example 4

The alcohol compound (IV) (1.0790 g, 2.76 mmol) was dissolved in diethyl ether (9 ml) and cooled with ice-water to 0°C. Then, pyridine (15 mg) and PBr₃ (92 µL, 263 mg, 0.97 mmol) were added thereto and the mixture was stirred for 2.5 hours. After completion of the reaction, extraction was performed by addition of water and AcOEt and the resultant organic layer was washed with saturated saline, dried over MgSO₄ and concentrated to obtain the halogen compound (V) (1.1869 g, 2.62 mmol, yield: 95%).
¹H-NMR: δ (CDCl₃) 1.00 (3H), 1.26 (3H), 1.33 (3H), 1.60-2.10 (2H), 2.17 (3H), 2.47 (3H), 2.30-2.60 (2H), 2.65-2.75 (1H), 2.95-3.20 (1H), 3.75-3.90 (2H), 4.00-4.25 (1H), 5.30-5.65 (1H), 7.30-7.45 (2H), 7.70-7.90 (2H)

### Example 5

The halogen compound (V) (1.1200 g, 2.47 mmol) was dissolved in anhydrous acetone (20 ml) and PPh₃ (647.9 mg, 2.47 mmol) were added thereto. The mixture was heated under reflux for 7.5 hours. After completion of the reaction, the reaction mixture was concentrated to obtain the phosphonium salt (VI) (1.8891 g).
¹H-NMR: δ (CDCl₃) 0.90-1.10 (3H), 1.10-1.20 (3H), 1.25 (3H), 1.20-1.40 (2H), 1.60-1.90 (2H), 2.10-2.20 (3H), 2.46 (3H), 2.30-2.60 (1H), 2.90-3.20 (1H), 4.00-4.20 (1H), 4.30-4.90 (2H), 5.10-5.60 (1H), 7.20-7.90 (19H)

### Reference Example 1

The alcohol compound (VII) (1.8945 g, 5 mmol) was dissolved in diethyl ether (5 ml) and, after cooling with ice-water to 0°C, pyridine (25 mg) and PBr₃ (158 µL, 451 mg, 1.67 mmol) were added thereto and the mixture was stirred for 3.5 hours. Two drops of PBr₃ were added by a pipette and the mixture was further stirred for 4 hours. After completion of the reaction, extraction was performed by addition of water and Et₂O and the organic layer was washed with saturated saline, dried over MgSO₄ and concentrated to obtained the halogen compound (VIII) (2.083 g, 4.53 mmol, yield: 95%).

### Example 6

The halogen compound (VIII) (883 mg, 2 mmol) was dissolved in anhydrous acetone (15 ml), PPh₃ (524.6 mg, 2 mmol) was added thereto and the mixture was stirred at room temperature for 1.5 hours. Then, the temperature was raised to 40°C and the mixture was stirred for 15 hours and further heated under reflux for 5 hours. After completion of the reaction, the reaction mixture was concentrated and washed with hexane, and diethyl ether was added thereto. After separation into two layers, the diethyl ether layer was separated and the residual solution was concentrated to obtain a crude product (1.4572 g). According to NMR analysis, the product was the phosphonium salt (IX) containing some impurities.
¹H-NMR: δ (CDCl₃) 0.72-0.87 (6H), 1.26 (3H), 1.15-1.60 (5H), 1.80-2.10 (1H), 2.17-2.19 (3H), 2.44 (3H), 2.30-2.50 (1H), 3.00-3.20 (1H), 3.80-3.90 (1H), 4.30-4.90 (2H), 5.10-5.50 (1H), 7.20-7.90 (19H)

### Example 7

The C10 dialdehyde (X) (172. 4 mg, 1.05 mmol) was dissolved in CH₂Cl₂ (3 ml) and an aqueous 2 M NaOH solution (2 ml) was added thereto at room temperature. To the resultant solution was added dropwise a solution of the phosphonium compound (VI) dissolved in CH₂Cl₂ in an amount corresponding to 2.29 mmol and then the mixture was stirred for 7 hours. After completion of the reaction, the aqueous layer was separated and the organic layer was washed several times with water. After drying over MgSO₄, the layer was concentrated and purified by silica gel column chromatography to obtain the disulfone compound (XI) (625.5 mg, 68%) and the monoaldehyde (XII) (108.0 mg, 20%).

### Disulfone compound (XI)

¹H-NMR: δ (CDCl₃) 0.90 (6H), 1.28 (6H), 1.30-2.00 (18H), 2. 05 (6H), 2.20 (6H), 2.45 (6H), 2.35-2.60 (4H), 2. 60-2. 80 (2H), 3. 00-3.20 (2H), 5.80-6.00 (1H), 6.10-6.30 (2H), 6. 30-6. 70 (1H), 7.20-7.40 (4H), 7.60-7.90 (4H)

### Example 8

The C10 dialdehyde (X) (82 mg, 0.5 mmol) was dissolved in CH₂Cl₂ (1.5 ml) and an aqueous 2 M NaOH solution (1.5 ml) was added thereto at room temperature. To the resultant solution was added dropwise a CH₂Cl₂ solution of the phosphonium salt (IX) in an amount corresponding to 1 mmol and then the mixture was stirred for 10 hours. After completion of the reaction, the aqueous layer was separated and the organic layer was washed several times with water. The organic layer was dried over MgSO4 and concentrated and the product was isolated and purified by silica gel chromatography to obtain the disulfone compound (XIII) (0.41588g, 98%).
¹H-NMR: δ (CDCl₃) 0.80-0.83 (6H), 1.05-1.09 (6H), 1.30-1.70 (16H), 1.80-2.20 (18H), 2.43-2.44 (6H), 2.60-2.80 (2H), 2.90-3.20 (2H), 3.60-4.00 (4H), 5.80-6.00 (1H), 6.10-6.80 (3H), 7.20-7.40 (4H), 7.60-7.90 (4H)

### Example 9

A mixture of the disulfone compound (XI) and the monoaldehyde (XII) (343. 6mg, containing the disulfone compound (XI) (273.9 mg, 0.31 mmol)) was dissolved in THF (2 ml), KOMe (137 mg, 1.95 mmol) was added thereto and the mixture was stirred at 40°C for 7.5 hours. After completion of the reaction, THF was distilled off and extraction was performed by addition of CHCl₃ and water to the concentrate. The extract was dried over MgSO₄, concentrated and purified by silica gel column chromatography to obtain canthaxanthin (72.8 mg, 0.129 mmol, yield: 41.3%).

### Example 10

The disulfone compound (XIII) (346.9 mg, 0.4 mmol) was dissolved in THF (2 ml). To the solution was added KOMe (112 mg, 1.6 mmol) and the mixture was stirred at 40°C for 4.5 hours. After completion of the reaction, THF was distilled off and the concentrate was isolated by silica gel chromatography to obtain β-carotene (141.1 mg, 0.263 mmol, yield: 66%).

Chemical structures of the compounds used in Examples are shown below:

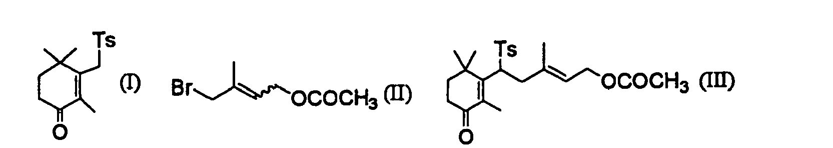

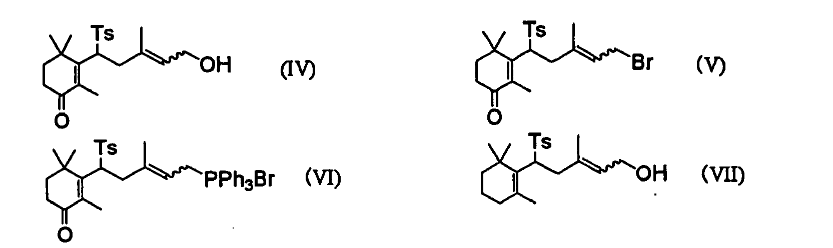

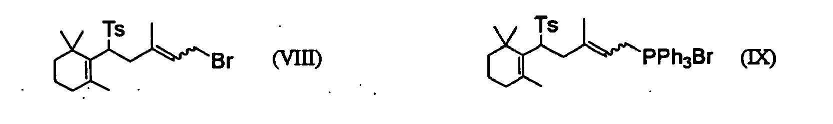

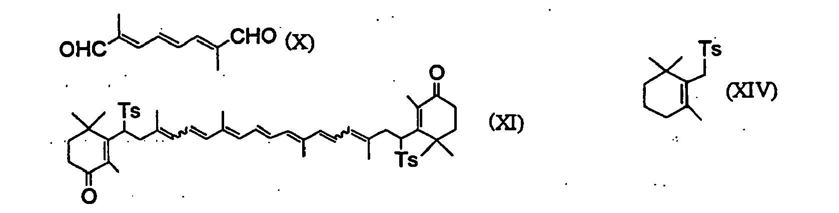

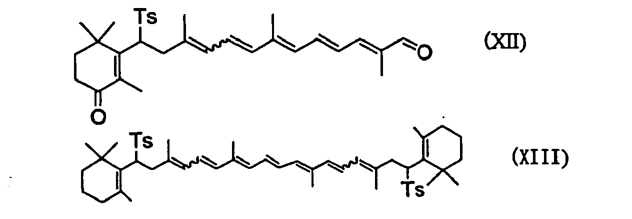

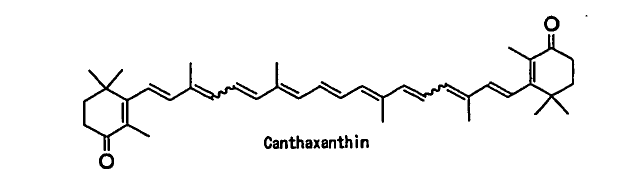

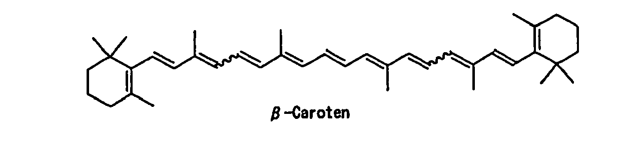

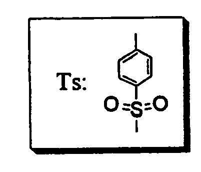

### Industrial Applicability

By using the process of the present invention, carotenoids which are important in the fields of medicine, feed additives and food additives can be prepared from readily available myrcene and linalool through relatively stable intermediates, industrially and advantageously.

## Claims

1. A disulfone compound represented by the general formula (3): 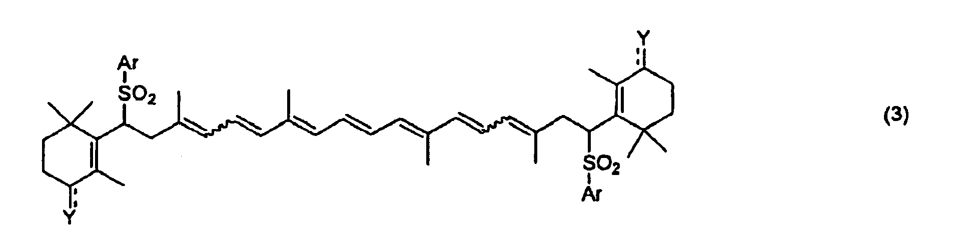 wherein Ar is an optionally substituted aryl group; Y is a hydrogen atom or an oxo group; and each wavy line represents either of E/Z geometrical isomers or a mixture thereof.

2. A ketosulfone compound represented by the general formula (1): 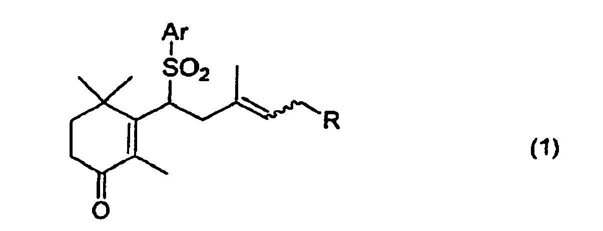 wherein R is a hydroxy group, -OCOR' group or a halogen, wherein R' is a straight- or branched-chain C₁-C₅ alkyl group; and Ar and the wavy line are as defined above.

3. A phosphonium salt represented by the general formula (2): 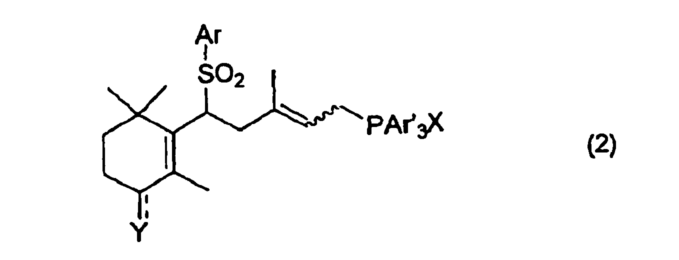 wherein each of Ar and Ar' is an optionally substituted aryl group; X is a halogen or -OSO₂R" group, wherein R" is an optionally substituted aryl group, a straight- or branched-chain C₁-C₅ alkyl group or a trifluoromethyl group; and Y and the wavy line are as defined above.

4. A process for preparation of the disulfone compound represented by the above general formula (3), which comprises reacting the phosphonium salt represented by the above general formula (2) with a C10 dialdehyde represented by the formula (5) : 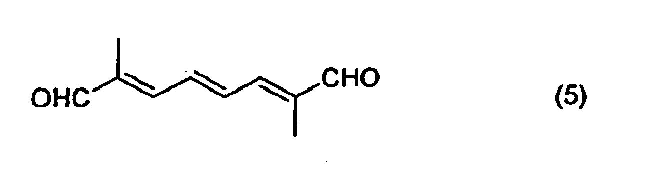 in the presence of a base.

5. A process for preparation of the disulfone compound represented by the above general formula (3) according to claim 4, which comprises reacting a sulfone compound represented by the general formula (6): 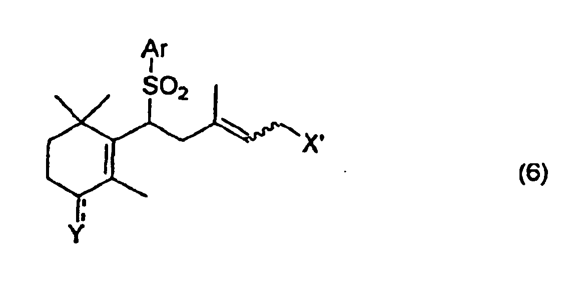 wherein X' is a hydroxy group or a halogen; and Ar, Y and the wavy line are as defined above, with a triarylphosphine represented by the general formula (7):
PAr'3 (7)
wherein Ar' is as defined above, a hydrohalogenic acid salt or sulfonate thereof, followed by reacting the resultant phosphonium salt represented by the above general formula (2) with the C₁₀ dialdehyde represented by the above formula (5) in the presence of a base.

6. A process for preparation of the phosphonium salt represented by the above general formula (2), which comprises reacting the sulfone compound represented by the above general formula (6) with the triarylphosphine represented by the above general formula (7) or a hydrohalogenic acid salt or sulfonate thereof.

7. A process for preparation of an ester compound represented by the general formula (11): 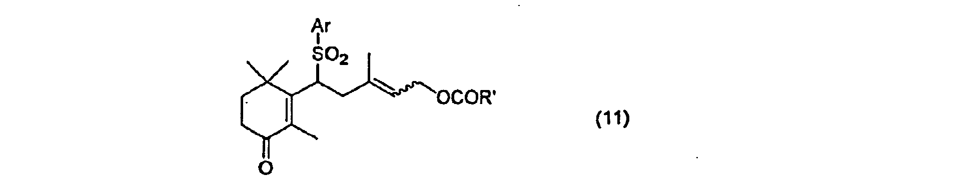 wherein Ar, R' and the wavy line are as defined above; which comprises reacting a cyclic ketosulfone compound represented by the general formula (9): 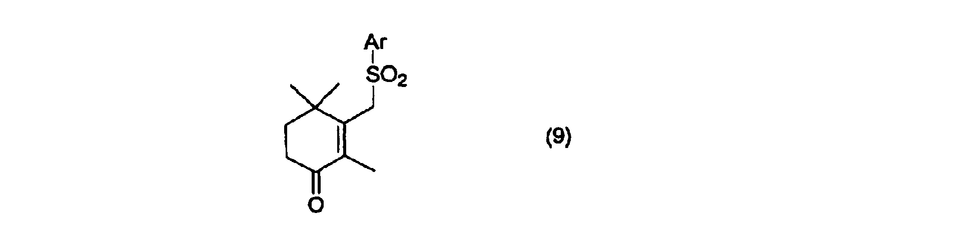 wherein Ar is as defined above, with an allyl halide represented by the general formula (10): 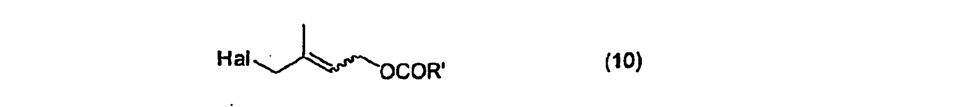 wherein Hal is a halogen; R' is a straight- or branched-chain C₁-C₅ alkyl group; and the wavy line is as defined above, in the presence of a base.

8. A process for preparation of an alcohol compound represented by the general formula (12): 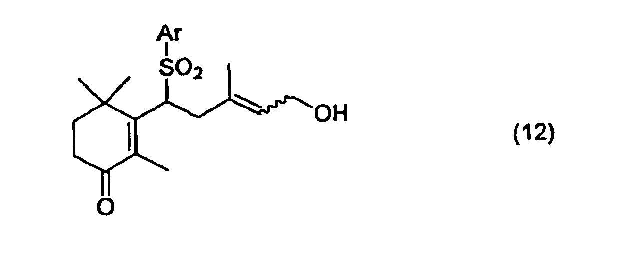 wherein Ar and the wavy line are as defined above, which comprises hydrolyzing the ester compound represented by the above general formula (11).

9. A process for preparation of the alcohol compound represented by the above general formula (12) according to claim 8, which comprises reacting the cyclic ketosulfone compound represented by the above general formula (9) with the allyl halide represented by the above general formula (10) in the presence of a base, followed by hydrolyzing the resultant ester compound represented by the above general formula (11).

10. A process for preparation of a halogen compound represented by the general formula (13): 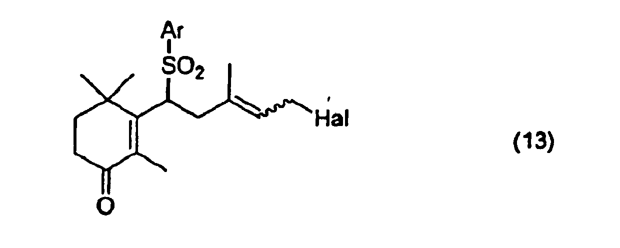 wherein Ar, Hal and the wavy line are as defined above, which comprises reacting the alcohol compound represented by the above general formula (12) with a halogenating agent.

11. A process for preparation of the halogen compound represented by the above general formula (13) according to claim 10, which comprises reacting the alcohol compound represented by the above general formula (12), which has been obtained by hydrolysis of the ester compound represented by the above general formula (11), with a halogenating agent.

12. The process for preparation of the halogen compound according to claim 11, wherein the ester compound represented by the above general formula (11) is a compound obtained by reacting the ketosulfone compound represented by the above general formula (9) with the allyl halide represented by the above general formula (10) in the presence of a base.

13. A process for preparation of a carotenoid represented by the general formula (4): 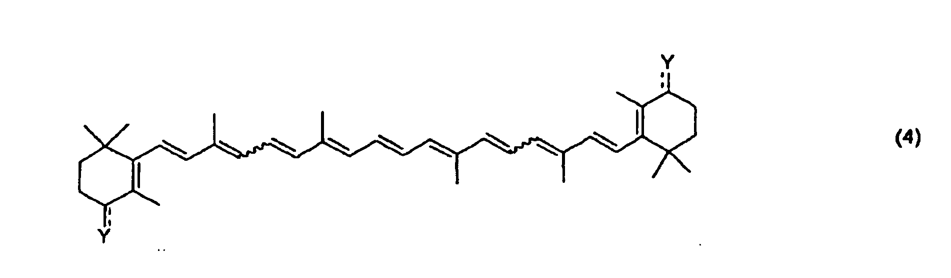 wherein Y and each wavy line are as defined above, which comprises reacting the disulfone compound represented by the above general formula (3) with a basic compound.

14. A process for preparation of the carotenoid represented by the above general formula (4) according to claim 13, which comprises reacting the phosphonium salt represented by the above general formula (2) with the C10 dialdehyde represented by the above formula (5) in the presence of a base, followed by reacting the resulting disulfone compound represented by the above general formula (3) with a basic compound.

15. A process for preparation of the carotenoid represented by the above general formula (4) according to claim 14, which comprises reacting the sulfone compound represented by the above general formula (6) with the triarylphosphine represented by the above general formula (7) or a hydrohalogenic acid salt or sulfonate thereof, reacting the resultant phosphonium salt represented by the above general formula (2) with the C10 dialdehyde represented by the above formula (5) in the presence of a base, and reacting the resultant disulfone compound represented by the above general formula (3) with a basic compound.

## Patentansprüche

1. Disulfonverbindung der allgemeinen Formel (3): 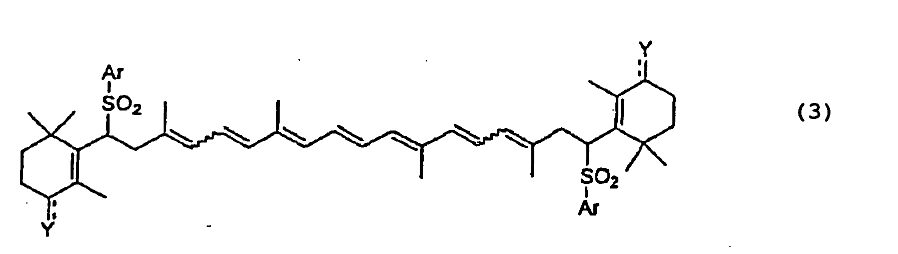 worin Ar für eine optional substituierte Arylgruppe steht; Y für ein Wasserstoffatom oder eine Oxogruppe steht; und jede Wellenlinie für eines der geometrischen E/Z-Isomere oder ein Gemisch derselben steht.

2. Ketosulfonverbindung der allgemeinen Formel (1): 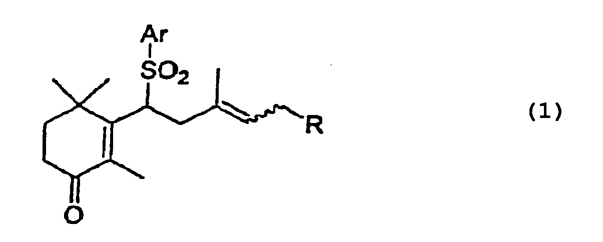 worin R für eine Hydroxygruppe, eine -OCOR'-Gruppe oder ein Halogen steht, wobei R' für eine geradkettige oder verzweigkettige C₁-C₅-Alkylgruppe steht; und Ar und die Wellenlinie wie im vorhergehenden definiert sind.

3. Phosphoniumsalz der allgemeinen Formel (2): 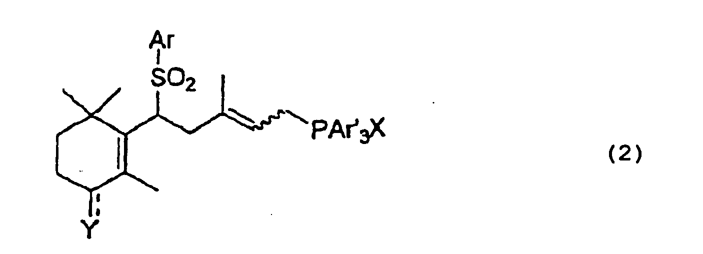 worin Ar und Ar' jeweils für eine optional substituierte Arylgruppe stehen; X für ein Halogen oder eine -OSO₂R"-Gruppe steht, wobei R" für eine optional substituierte Arylgruppe, eine geradkettige oder verzweigtkettige C₁-C₅-Alkylgruppe oder eine Trifluormethylgruppe steht; und Y und die Wellenlinie wie im vorhergehenden definiert sind.

4. Verfahren zur Herstellung der Disulfonverbindung der obigen allgemeinen Formel (3), wobei das Verfahren das Umsetzen des Phosphoniumsalzes der obigen allgemeinen Formel (2) mit einem C₁₀-Dialdehyd der Formel (5): 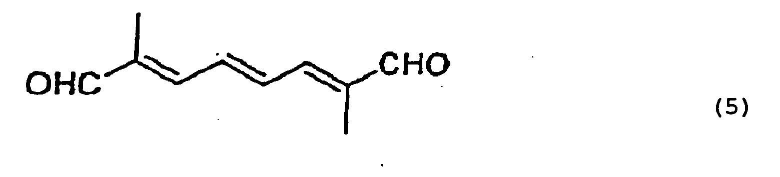 in Gegenwart einer Base umfasst.

5. Verfahren zur Herstellung der Disulfonverbindung der obigen allgemeinen Formel (3) nach Anspruch 4, wobei das Verfahren das Umsetzen einer Sulfonverbindung der allgemeinen Formel (6): 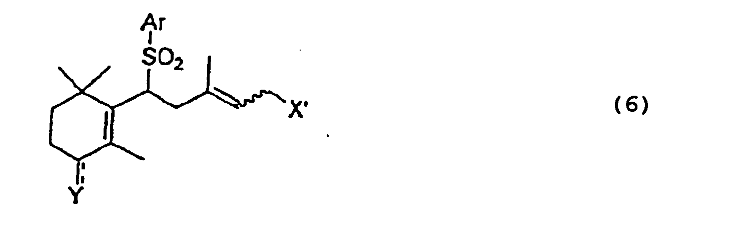 worin X' für eine Hydroxygruppe oder ein Halogen steht; und Ar, Y und die Wellenlinie wie im vorhergehenden definiert sind,
mit einem Triarylphosphin der allgemeinen Formel (7):
PAr'₃ (7)
worin Ar' wie im vorhergehenden definiert ist,
einem Salz einer Halogenwasserstoffsäure oder einem Sulfonat hiervon,
und das nachfolgende Umsetzen des erhaltenen Phosphoniumsalzes der obigen allgemeinen Formel (2) mit dem C₁₀-Dialdehyd der obigen allgemeinen Formel (5) in Gegenwart einer Base umfasst.

6. Verfahren zur Herstellung des Phosphoniumsalzes der obigen allgemeinen Formel (2), wobei das Verfahren das Umsetzen der Sulfonverbindung der obigen allgemeinen Formel (6) mit dem Triarylphosphin der obigen allgemeinen Formel (7) oder einem Salz einer Halogenwasserstoffsäure oder einem Sulfonat hiervon umfasst.

7. Verfahren zur Herstellung einer Esterverbindung der allgemeinen Formel (11): 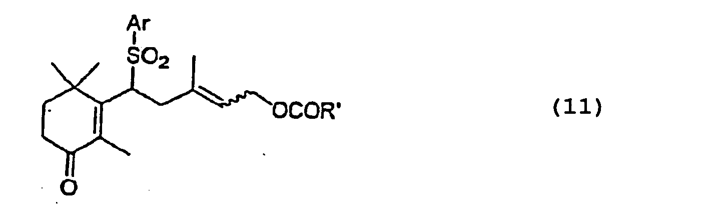 worin Ar, R' und die Wellenlinie wie im vorhergehenden definiert sind;
wobei das Verfahren das Umsetzen einer cyclischen Ketosulfonverbindung der allgemeinen Formel (9): 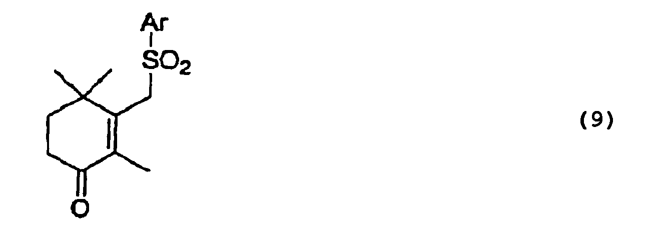 worin Ar wie im vorhergehenden definiert ist,
mit einem Allylhalogenid der allgemeinen Formel (10): 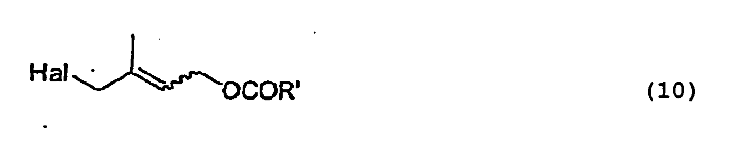 worin Hal für ein Halogen steht; R' für eine geradkettige oder verzweigtkettige C₁-C₅-Alkylgruppe steht; und die Wellenlinie wie im vorhergehenden definiert ist,
in Gegenwart einer Base umfasst.

8. Verfahren zur Herstellung einer Alkoholverbindung der allgemeinen Formel (12): 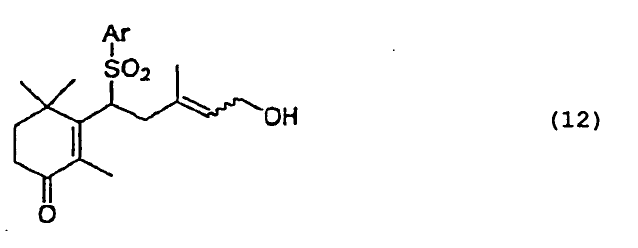 worin Ar und die Wellenlinie wie im vorhergehenden definiert sind,
wobei das Verfahren das Hydrolysieren der Esterverbindung der obigen allgemeinen Formel (11) umfasst.

9. Verfahren zur Herstellung der Alkoholverbindung der obigen allgemeinen Formel (12) nach Anspruch 8, wobei das Verfahren das Umsetzen der cyclischen Ketosulfonverbindung der obigen allgemeinen Formel (9) mit dem Allylhalogenid der obigen allgemeinen Formel (10) in Gegenwart einer Base und das nachfolgende Hydrolysieren der erhaltenen Esterverbindung der obigen allgemeinen Formel (11) umfasst.

10. Verfahren zur Herstellung einer Halogenverbindung der allgemeinen Formel (13): 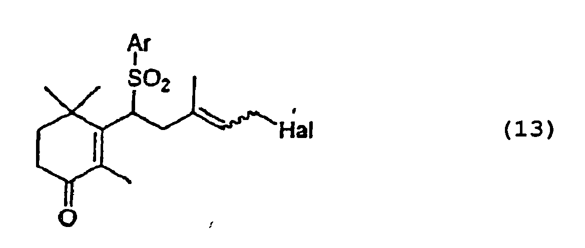 worin Ar, Hal und die Wellenlinie wie im vorhergehenden definiert sind,
wobei das Verfahren das Umsetzen der Alkoholverbindung der obigen allgemeinen Formel (12) mit einem Halogenierungsmittel umfasst.

11. Verfahren zur Herstellung der Halogenverbindung der obigen allgemeinen Formel (13) nach Anspruch 10, wobei das Verfahren das Umsetzen der Alkoholverbindung der obigen allgemeinen Formel (12), die durch Hydrolyse der Esterverbindung der obigen allgemeinen Formel (11) erhalten wurde, mit einem Halogenierungsmittel umfasst.

12. Verfahren zur Herstellung der Halogenverbindung nach Anspruch 11, wobei die Esterverbindung der obigen allgemeinen Formel (11) eine Verbindung ist, die durch Umsetzen der Ketosulfonverbindung der obigen allgemeinen Formel (9) mit dem Allylhalogenid der obigen allgemeinen Formel (10) in Gegenwart einer Base erhalten wird.

13. Verfahren zur Herstellung eines Carotinoids der allgemeinen Formel (4): 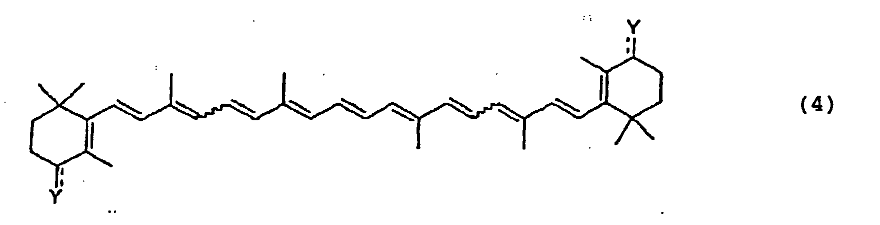 worin Y und jede Wellenlinie wie im vorhergehenden definiert sind,
wobei das Verfahren das Umsetzen der Disulfonverbindung der obigen allgemeinen Formel (3) mit einer basischen Verbindung umfasst.

14. Verfahren zur Herstellung des Carotinoids der obigen allgemeinen Formel (4) nach Anspruch 13,
wobei das Verfahren das Umsetzen des Phosphoniumsalzes der obigen allgemeinen Formel (2) mit dem C₁₀-Dialdehyd der
obigen Formel (5) in Gegenwart einer Base und das nachfolgende Umsetzen der erhaltenen Disulfonverbindung der obigen allgemeinen Formel (3) mit einer basischen Verbindung umfasst.

15. Verfahren zur Herstellung des Carotinoids der obigen allgemeinen Formel (4) nach Anspruch 14,
wobei das Verfahren das Umsetzen der Sulfonverbindung der obigen allgemeinen Formel (6) mit dem Triarylphosphin der obigen allgemeinen Formel (7) oder einem Salz einer Halogenwasserstoffsäure oder einem Sulfonat hiervon, das Umsetzen des erhaltenen Phosphoniumsalzes der obigen allgemeinen Formel (2) mit dem C₁₀-Dialdehyd der obigen Formel (5) in Gegenwart einer Base und das Umsetzen der erhaltenen Disulfonverbindung der obigen allgemeinen Formel (3) mit einer basischen Verbindung umfasst.

## Revendications

1. Composé disulfone représenté par la formule générale (3) : 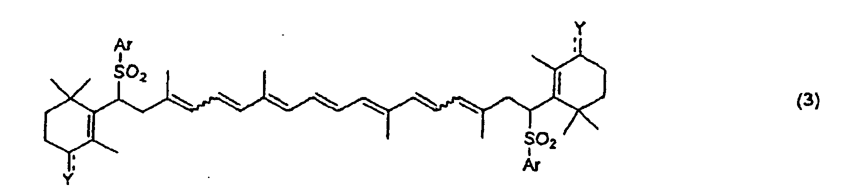 dans laquelle Ar est un groupe aryle éventuellement substitué ; Y est un atome d'hydrogène ou un groupe oxo ; et chaque trait ondulé représente l'un ou l'autre des isomères géométriques E/Z ou un mélange de ceux-ci.

2. Composé cétosulfone représenté par la formule générale (1) : 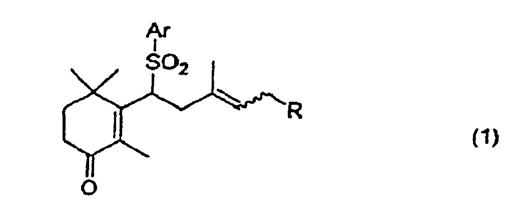 dans laquelle R est un groupe hydroxy, un groupe -OCOR' ou un halogène, où R' est un groupe alkyle en C₁ à C₅ à chaîne linéaire ou ramifiée ; et Ar et le trait ondulé sont tels que définis ci-dessus.

3. Sel de phosphonium représenté par la formule générale (2) : 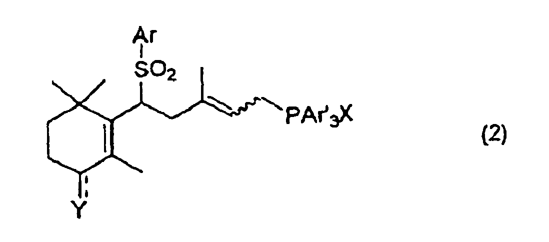 dans laquelle chacun des Ar et Ar' est un groupe aryle éventuellement substitué ; X est un halogène ou un groupe -OSO₂R", où R" est un groupe aryle éventuellement substitué, un groupe alkyle en C₁ à C₅ à chaîne linéaire ou ramifiée ou un groupe trifluorométhyle ; et Y et le trait ondulé sont tels que définis ci-dessus.

4. Procédé pour la préparation du composé disulfone représenté par la formule générale (3) ci-dessus, qui comprend la réaction du sel de phosphonium représenté par la formule générale (2) ci-dessus avec un dialdéhyde en C₁₀ représenté par la formule (5) : 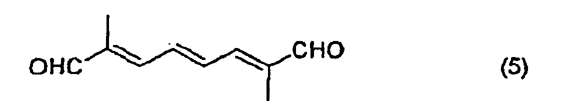 en présence d'une base.

5. Procédé pour la préparation du composé disulfone représenté par la formule générale (3) ci-dessus selon la revendication 4, qui comprend la réaction d'un composé sulfone représenté par la formule générale (6) : 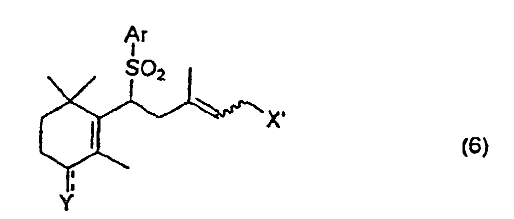 dans laquelle X' est un groupe hydroxy ou un halogène ; et Ar, Y et le trait ondulé sont tels que définis ci-dessus, avec une triarylphosphine représentée par la formule générale (7) :
PAr'3 (7)
dans laquelle Ar' est tel que défini ci-dessus, un sel d'acide halogénhydrique ou un sulfonate de celui-ci, suivie par la réaction du sel de phosphonium obtenu représenté par la formule générale (2) ci-dessus avec le dialdéhyde en C₁₀ représenté par la formule (5) ci-dessus en présence d'une base.

6. Procédé pour la préparation du sel de phosphonium représenté par la formule générale (2) ci-dessus, qui comprend la réaction du composé sulfone représenté par la formule générale (6) ci-dessus avec la triarylphosphine représentée par la formule générale (7) ci-dessus ou un sel d'acide halogénhydrique ou un sulfonate de celui-ci.

7. Procédé pour la préparation d'un composé ester représenté par la formule générale (11) : 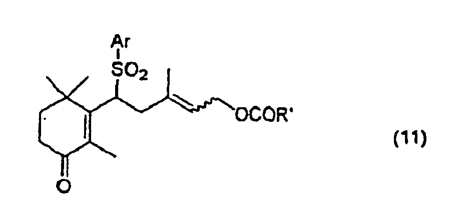 dans laquelle Ar, R' et le trait ondulé sont tels que définis ci-dessus ; qui comprend la réaction d'un composé cétosulfone cyclique représenté par la formule générale (9) : 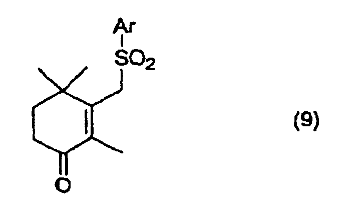 dans laquelle Ar est tel que défini ci-dessus, avec un halogénure d'allyle représenté par la formule générale (10) : 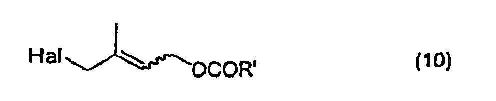 dans laquelle Hal est un halogène ; R' est un groupe alkyle en C₁ à C₅ à chaîne linéaire ou ramifiée ; et le trait ondulé est tel que défini ci-dessus, en présence d'une base.

8. Procédé pour la préparation d'un composé alcoolique représenté par la formule générale (12) : 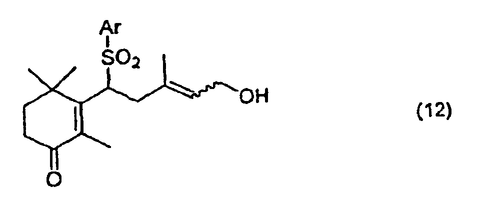 dans laquelle Ar et le trait ondulé sont tels que définis ci-dessus, qui comprend l'hydrolyse du composé ester représenté par la formule générale (11) ci-dessus.

9. Procédé pour la préparation du composé alcoolique représenté par la formule générale (12) ci-dessus selon la revendication 8, qui comprend la réaction du composé cétosulfone cyclique représenté par la formule générale (9) ci-dessus avec l'halogénure d'allyle représenté par la formule générale (10) ci-dessus en présence d'une base, suivie par l'hydrolyse du composé ester obtenu représenté par la formule générale (11) ci-dessus.

10. Procédé pour la préparation d'un composé halogéné représenté par la formule générale (13) : 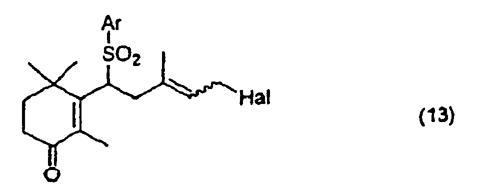 dans laquelle Ar, Hal et le trait ondulé sont tels que définis ci-dessus, qui comprend la réaction du composé alcoolique représenté par la formule générale (12) ci-dessus avec un agent d'halogénation.

11. Procédé pour la préparation du composé halogéné représenté par la formule générale (13) ci-dessus selon la revendication 10, qui comprend la réaction du composé alcoolique représenté par la formule générale (12) ci-dessus, qui a été obtenu par hydrolyse du composé ester représenté par la formule générale (11) ci-dessus, avec un agent d'halogénation.

12. Procédé pour la préparation du composé halogéné selon la revendication 11, dans lequel le composé ester représenté par la formule générale (11) ci-dessus est un composé obtenu en faisant réagir le composé cétosulfone représenté par la formule générale (9) ci-dessus avec l'halogénure d'allyle représenté par la formule générale (10) ci-dessus en présence d'une base.

13. Procédé pour la préparation d'un caroténoïde représenté par la formule générale (4) : 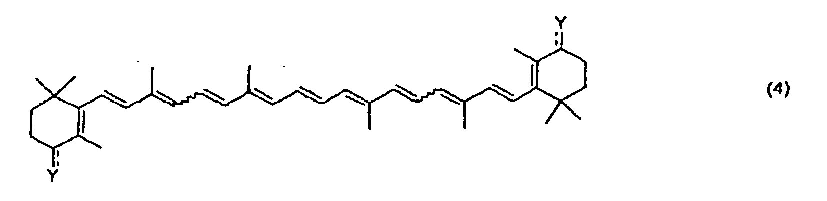 dans laquelle Y et chaque trait ondulé sont tels que définis ci-dessus, qui comprend la réaction du composé disulfone représenté par la formule générale (3) ci-dessus avec un composé basique.

14. Procédé pour la préparation du caroténoïde représenté par la formule générale (4) ci-dessus selon la revendication 13, qui comprend la réaction du sel de phosphonium représenté par la formule générale (2) ci-dessus avec le dialdéhyde en C₁₀ représenté par la formule (5) ci-dessus en présence d'une base, suivie par la réaction du composé disulfone obtenu représenté par la formule générale (3) ci-dessus avec un composé basique.

15. Procédé pour la préparation d'un caroténoïde représenté par la formule générale (4) ci-dessus selon la revendication 14, qui comprend la réaction du composé sulfone représenté par la formule générale (6) ci-dessus avec la triarylphosphine représentée par la formule générale (7) ci-dessus ou un sel d'acide halogénhydrique ou un sulfonate de celui-ci, la réaction du sel de phosphonium obtenu représenté par la formule générale (2) ci-dessus avec le dialdéhyde en C₁₀ représenté par la formule (5) ci-dessus en présence d'une base, et la réaction du composé disulfone obtenu représenté par la formule générale (3) ci-dessus avec un composé basique.
